# DEMANDE DE BREVET EUROPEEN

(11) **EP 4 026 557 A1**
(43) Date de publication de la demande: **13.07.2022**
(21) Numéro de dépôt: 21305014.9
(22) Date de dépôt: 06.01.2021
(51) Int. Cl.: A61K 38/46, A61P 15/00, C12Q 1/68

(54) **UTILISATION D'UNE DÉSOXYRIBONUCLÉASE I DANS LE TRAITEMENT DE L'ENDOMÉTRIOSE CHEZ DES PATIENTES PRÉSENTANT UNE SIGNATURE ÉPIGÉNÉTIQUE DE L'ENDOMÉTRIOSE SUR L'ADN ACELLULAIRE**

(71) Demandeur: Endolife, 75008 Paris (FR)
(72) Inventeur: HAZOUT, André, 75016 PARIS (FR); BEN KHALIFA, Moncef, 78320 LE MESNIL-SAINT-DENIS (FR)
(74) Mandataire: Santarelli

(57) **Abrégé**

L'invention porte sur une méthode de traitement de l'endométriose, par administration de DNase I, particulièrement pour traiter une patiente présentant un taux d'ADN acellulaire supérieur à la normale, a fortiori si cet ADN acellulaire présente un profil de méthylation typique de l'endométriose.

## Description

### DOMAINE DE L'INVENTION

La présente invention concerne le domaine du traitement de l'endométriose.

Plus particulièrement, l'invention propose un traitement par la désoxyribonucléase I (DNase I), permettant d'hydrolyser les acides désoxyribonucléiques libres (ADN acellulaire) en excès, chez des patientes souffrant de douleurs endométriosiques chroniques et présentant un certain profil de gènes hyper et hypo méthylés.

### ARRIERE-PLAN TECHNOLOGIQUE

### L'endométriose

L'endométriose est une maladie inflammatoire chronique, oestrogéno-dépendante, due à la migration des cellules de l'endomètre en-dehors de la cavité utérine, principalement au niveau des organes et des tissus pelviens. Cet état inflammatoire associé à des douleurs pelviennes et, parfois, à un état d'infertilité, atteint 3 à 10% des femmes jeunes en âge de procréer.

La maladie est hétérogène, allant de lésions superficielles péritonéales et séreuses à des kystes d'endométriose dans les ovaires (endométriome), des nodules dans l'endométriose profonde, et peut souvent être accompagnée de fibrose et d'adhérences. L'endométriose peut survenir chez une jeune fille pré-pubère algique (Marsh et Laufer 2005) puis de la puberté à la ménopause, où la cyclicité menstruelle et la douleur disparaissent.

Nous savons que la croissance des cellules endométriales ectopiques est dépendante des œstrogènes. L'endométriose est largement décrite comme une régurgitation du sang des règles avec une migration des cellules de l'endomètre vers les organes avoisinants et au-delà : abdomen, poumons, foie et cerveau.

Très rarement, une endométriose a également été constatée dans le tractus génito-urinaire de l'homme (Rei et Feloney, 2018). Vingt-deux cas d'endométrioses hépatiques ont aussi été publiés (Liu *et al.,* 2015). Ceci est un argument contre la théorie prédominante du flux menstruel rétrograde. Seulement 10% des femmes développent une endométriose alors que la régurgitation des règles survient dans 76% à 90% des femmes en âge de procréer (Blumenkrantz *et al.,* 1981 ; Halme *et al*., 1984).

Comme rappelé plus haut, l'endométriose est une maladie caractérisée par la présence de cellules endométriales ectopiques. Dans les formes superficielles, les moins graves, ces cellules migrent et se fixent sur le péritoine. Dans les formes plus sévères, les cellules gagnent différents organes : l'ovaire, la vessie ou encore la paroi intestinale. Cette localisation anormale est associée à une inflammation importante. Elle provoque des douleurs, en particulier au moment des règles, et parfois une infertilité. Une chirurgie est alors souvent nécessaire pour supprimer les nodules.

Les femmes souffrant d'une forme profonde de la maladie présentent un stress oxydatif majeur au niveau du péritoine. Ce stress s'accompagne d'une forte augmentation de fragments d'ADN libre dans le sang circulant (Zachariah *et al* 2004).

A ce stade il est difficile de dire si l'endométriose est à l'origine de ce stress ou si c'est l'inverse. Mais des expériences conduites in vitro montrent que le fait d'inhiber ce stress oxydatif bloque la prolifération des cellules endométriales (Filev *et al* 2019).

40% à 90% des femmes atteintes d'endométriose souffrent de douleurs. Le symptôme majeur est une douleur pelvienne récurrente parfois très aigüe, notamment au moment des règles. Les lésions sont en effet sensibles aux œstrogènes et vont donc proliférer, saigner et laisser des cicatrices fibreuses à chaque cycle menstruel. En dehors de la période des règles, les patientes peuvent également souffrir lors des rapports sexuels (dyspareunie) ou encore lorsqu'elles urinent ou évacuent leurs selles (Mc Peak *et al.* 2018).

La maladie peut, rarement, être totalement asymptomatique. Dans ce cas, elle est découverte de façon fortuite alors que la patiente consulte en raison d'une infertilité. L'explication scientifique de ce lien n'est pas entièrement élucidée. Des études récentes montrent par ailleurs que chez ces patientes, l'endomètre présente des profils hormonaux et l'expression de gènes anormaux (Naqvi *et al* 2014 ; Laganà *et al* 2019 ; Vassilopoulou *et al* 2019)

Cependant, alors que les cliniciens estiment que 90% des femmes présentent des saignements rétrogrades, 10% d'entre elles seulement, développent des lésions d'endométriose confirmée par laparoscopie.

L'endométriose a non seulement des effets physiques mais aussi psychologiques, provoquant la dépression, l'anxiété et compromettant les relations sociales. De plus, l'endométriose a un impact négatif sur la vie sexuelle et les rapports sociaux (Della Corte *et al* 2020).

### Diagnostic de l'endométriose

Le diagnostic d'endométriose est posé lors d'une laparoscopie et confirmé par l'analyse des lésions extraites. Le traitement est souvent médical pour les formes légères à modérées (stades I et II selon le score AFS révisé, ou score ASRM) et chirurgical immédiatement ou après une hormonothérapie pour les formes sévères (stades III et IV selon le score AFS révisé, ou score ASRM - *American Society of Reproductive Medicine*).

Selon une autre classification, issue des Recommandations pour la Pratique Clinique de l'endométriose (RPC endométriose) publiées par la Haute autorité de santé et le Collège national des gynécologues et obstétriciens de France (CNGOF) en 2018, on distingue 3 types d'endométriose :
- l'endométriose superficielle (ou péritonéale) qui désigne la présence d'implants d'endomètre ectopiques localisés à la surface du péritoine,
- l'endométriose ovarienne : l'endométriome ovarien est un kyste de l'ovaire caractérisé par son contenu liquidien couleur chocolat, et
- l'endométriose pelvienne profonde (ou sous-péritonéale) correspond aux lésions qui s'infiltrent en profondeur à plus de 5 mm sous la surface du péritoine. L'endométriose profonde peut toucher typiquement les ligaments utérosacrés (50 % des cas), le cul-de-sac vaginal postérieur (15 %), l'intestin (20-25 %), représenté majoritairement par la face antérieure du rectum et la jonction recto-sigmoïdienne, la vessie (10 %), les uretères (3 %) et au-delà de la cavité pelvienne, le sigmoïde, le côlon droit, l'appendice et l'iléon terminal pour les localisations les plus fréquentes.

L'endométriose a été décrite alternativement comme une maladie hormonale ou immunitaire, et une maladie génétique déclenchée par l'exposition à des facteurs environnementaux. En outre, plusieurs études suggèrent diverses aberrations épigénétiques dans la pathogenèse de l'endométriose (Koninck *et al.* 2019).

L'hétérogénéité du phénotype de la maladie est authentifiée par un grand nombre de laparoscopies faussement négatives chez la femme symptomatique. Aussi les examens anatomopathologiques, immuno-histochimiques et épigénétiques des lésions ne se sont pas révélées fiables (Ahn *et al.,* 2017), notamment concernant la méthylation de l'ADN génomique du récepteur B de la progestérone, de l'e-cadherine, de l'homeobox A10 (HOXA10), du récepteur d'oestrogène b, du facteur stéroïdogène 1(SF1) et de l'aromatase.

L'expression aberrante de l'ADN méthyltransférase, qui adjoint un groupe méthyle en position 5 des bases cytosines dans l'îlot CpG (Cytosine phosphate Guanine) du promoteur du gène, rendant silencieuse l'expression génique correspondante, a été démontrée dans l'endométriose (Nasu *et al,* 2011).

### Traitement de l'endométriose

Lorsqu'une patiente est algique, on lui propose, le plus souvent, en première intention un traitement hormonal destiné à supprimer les règles (contraceptifs œstroprogestatifs monophasiques en continu, progestatifs, Danazol ou analogues de la GnRH). Ce traitement réduit les douleurs par son effet antigonadotrope. De plus ces traitements, en période d'activité génitale, empêchent la réalisation d'un projet parental.

### ADN acellulaire

Dans le sang humain, la présence d'ADN libre circulant sans cellules (cfDNA) a été rapportée en 1948 (Mandel et Metais en1948). Le cfDNA a été étudié dans un large éventail de conditions physiologiques et pathologiques, notamment les troubles inflammatoires, le stress oxydatif, la stérilité du couple (EP2879696B1) et les tumeurs malignes.

Chez les individus sains, lors de la phagocytose, les corps apoptotiques ou nécrotiques sont ingérés par les macrophages. Ainsi, le phénomène de relargage de l'ADN libre ne peut pas avoir lieu. En revanche, lorsque les fragments d'ADN subsistent dans les nucléosomes qui sont relargués par les macrophages, ils sont protégés de la dégradation enzymatique et ainsi restent dans la circulation sanguine.

Le cfDNA est composé d'acides nucléiques double brins au poids moléculaire plus faible que l'ADN génomique. La taille de ces fragments génomiques est variable, allant pour les plus courts de 70 à 200 pb et pour les plus longs jusqu'à 21 kb. Le cfDNA est présent chez le sujet sain à des concentrations sanguines évaluées entre 50 et 250 ng/ml (EP2879696B1).

Les mécanismes biologiques de libération de l'ADN libre dans le sang ne sont pas entièrement connus. Des fragments d'ADN libre peuvent provenir de cellules nécrotiques englouties par les macrophages qui se libèrent alors partiellement. Selon cette hypothèse, les niveaux de cfDNA devraient être corrélés à l'étendue de la nécrose et/ou l'apoptose cellulaire (Grabushnig *et al.* 2020)

La clairance du cfDNA de la circulation sanguine est rapide (demi-vie : 16,3 min.); le cfDNA est sensible aux nucléases plasmatiques, mais les clairances rénales et hépatiques participent également à son élimination.

Le cfDNA peut être isolé du plasma et du sérum, mais le sérum contient une concentration d'ADN environ 6 fois supérieure (incorporation dans les cellules).

Les niveaux d'ADN et les schémas de fragmentation offrent des possibilités intéressantes à des fins diagnostiques, pronostiques et thérapeutiques.

Une étude de Zachariah (2009) a montré un cfDNA nucléaire et mitochondrial significativement plus élevé dans un groupe de femmes atteintes d'endométriose minime à légère que dans un groupe témoin (p = 0,046). Le seuil à partir d'une courbe ROC montrait une sensibilité à 70% et une spécificité à 87%. L'auteur concluait que le cfDNA circulant pouvait constituer un biomarqueur potentiel de l'endométriose minime et légère.

### L'endonucléase I (DNase I) :

La DNase I est l'enzyme responsable de la digestion de l'ADN extracellulaire.

Elle catalyse les acides désoxyribonucléiques en nucléotides ou polynucléotides

Elle hydrolyse les liaisons phosphodiester et réalise une coupure, de préférence sur les bases pyrimidiques. En présence d'ions manganèse elle coupe les deux brins en bouts francs. Avec des ions magnésium, elle coupe un brin en petits fragments.

Ses capacités à hydrolyser l'ADN sont couramment exploitées en biochimie, par exemple pour détecter l'empreinte des protéines fixées sur un génome cellulaire. En médecine, la forme recombinante humaine de la DNase I est, sous le nom de Pulmozyme^{®}, le plus puissant mucolytique utilisé dans le traitement permanent et quotidien de la mucoviscidose.

Les données montrées dans le brevet EP2879696B1, portant sur l'utilisation de la DNase I chez des femmes présentant une infertilité sans cause identifiée (notamment, sans diagnostic d'endométriose), avaient montré qu'une injection de 2500 UI de DNase I, sur une courte durée, diminuait de 40% le taux d'ADN libre circulant.

A ce jour, il n'existe aucune étude montrant l'efficacité de la DNase I sur la douleur et l'inflammation d'une endométriose modérée à sévère.

### RESUME DE L'INVENTION

La présente invention porte sur l'utilisation de DNase pour traiter l'endométriose.

En particulier, la présente invention vise à réduire l'inflammation et la douleur chez des patientes souffrant d'endométriose avec un taux d'ADN libre élevé, un profil de gènes candidats et un différentiel de méthylation évocateurs, en utilisant une DNAse I exogène et en maintenant voire favorisant la possibilité d'une grossesse.

### DESCRIPTION DETAILLEE

Dans une étude préalable, les inventeurs ont mis en évidence la pertinence du taux d'ADN acellulaire dans le diagnostic de l'endométriose, ainsi qu'un profil épigénétique de l'endométriose (demande de brevet PCT/EP2020/069015, non publiée avant le dépôt de la présente demande).

En effet, les inventeurs ont montré que : (i) une hyperméthylation des gènes sélectionnés parmi CALD1, RRP1, FN1, FAM87B, TCEAL6, RPL29P2, ATP11A-AS1, DIP2C, SLCO2B1, RMI2, MIR3170, LINC01007, TSPAN17, MIR4693, HYOU1, TLR4, ADGRL3, IL6, VIRMA, MKRN1, INSIG1, ROR2, MRPL3, FMNL2, TMEM19, ZNF438, LINC01192, RCBTB1, TSPAN33, NKD2, FGFR2, TPRG1, MIR4644, FOXO4, FSTL1 et CLMN, et/ou (ii) une hypométhylation des gènes sélectionnés parmi STAU2-AS1, TDRD5, USP1, ACVR2A, FBXO38, NT5C2, NAV1, SOD3, C3, UBE3A, MIR4655, MYO5C, COX6C, MIR6133, BRSK2, MIR4277, MIR4251, MN1, MIR3666, AZIN1, MIR4251, SLC37A2, FZD10, FASN, MKRN9P et PCCA-AS1, constituent des marqueurs d'une endométriose dont le potentiel évolutif est d'autant plus important que l'hyper- ou hypo-méthylation mesurée est importante.

Plus particulièrement, les inventeurs ont identifié une liste de 15 gènes fortement impliqués dans l'endométriose :
- (10 et 5), à savoir: CALD1, RRP1, FN1, FAM87B, TCEAL6, RPL29P2, ATP11A-AS1, DIP2C, SLCO2B1 et RMI2, hyperméthylés dans l'ADN acellulaire des femmes atteintes d'endométriose, et
- FBX038, ACVR2A, USP1, TDRD5 et STAU2-AS1, hypométhylés dans l'ADN acellulaire des femmes atteintes d'endométriose.

Poursuivant leurs recherches en vue de traiter les patientes atteintes d'endométriose, les inventeurs ont obtenu les résultats expérimentaux ci-dessous, qui apportent la preuve que l'endométriose peut être traitée en agissant sur l'ADN acellulaire des patientes, notamment en leur administrant de la DNase.

### Traitement de l'endométriose par l'administration de DNase

Selon un premier aspect, la présente invention porte sur l'utilisation d'une DNase, dans le traitement de l'endométriose.

Par « traitement », on entend ici toute réduction ou amélioration de la gravité et/ou de la progression de l'endométriose, en particulier la réduction d'un ou plusieurs symptômes de celle-ci, comme par exemple une réduction de l'inflammation et/ou de la douleur. Cette amélioration peut résulter de l'administration de la DNase seule, ou d'une thérapie associant la DNase à une autre thérapie (par exemple, une chirurgie pour les formes les plus graves).

Selon une mise en œuvre particulière de l'invention, la DNase administrée est une DNase recombinante.

Plus particulièrement, la présente invention peut être mise en œuvre en administrant de la DNase I, telle que la dornase alfa (Pulmozyme^{®}), actuellement indiquée dans le traitement de l'encombrement bronchique pour améliorer la fonction respiratoire chez des patients atteints de mucoviscidose.

### Sous-groupes de patientes

Dans une mise en œuvre particulière de l'invention, la DNase est utilisée dans le traitement de l'endométriose chez une patiente présentant un taux d'ADN acellulaire statistiquement supérieur au taux d'ADN libre chez les femmes n'ayant pas d'endométriose.

En pratique, l'homme du métier peut déterminer, par des études de routine sur des cohortes de patientes atteintes d'endométriose et de contrôles (par exemple, des femmes n'ayant pas d'endométriose), un ou plusieurs seuil(s) d'ADN acellulaire (éventuellement différents selon le type d'endométriose) à partir duquel le traitement par la DNase est particulièrement recommandé. Pour déterminer les patientes les plus susceptibles de répondre au traitement, l'ADN acellulaire peut être mesuré à partir d'un échantillon de fluide biologique de la patiente. A titre non limitatif de fluide biologique utilisable dans le cadre de l'invention, on peut citer le sang, le plasma et le sérum.

La quantification du cfDNA peut être réalisée comme indiqué dans la partie expérimentale ci-dessous, ou à l'aide d'autres méthodes décrites dans la littérature scientifique, notamment des méthodes fluorométriques ou spectrophotométriques telles que QUBIT^{®} (Life Technologies) ou NANODROP^{™} (Thermo Scientific). Récemment, l'analyse de l'ADN acellulaire a été abondamment décrite dans des méthodes de diagnostic de certains cancers ou dans le diagnostic prénatal d'anomalies chromosomiques. Aussi, plusieurs technologies pour isoler et analyser l'ADN acellulaire ont été décrites, tant dans les publications scientifiques que la littérature brevets. L'homme du métier peut parfaitement choisir, parmi les multiples technologies décrites, celle qui lui semble appropriée pour identifier les patientes les plus à même de bénéficier d'un traitement par la DNase.

Selon une autre mise en œuvre particulière de l'invention, la DNase est utilisée dans le traitement de l'endométriose chez une patiente dont l'ADN acellulaire présente un profil particulier ; en effet, comme mentionné ci-dessus, les inventeurs ont identifié que l'hyperméthylation de certains gènes et/ou l'hypométhylation de certains autres gènes, au niveau de l'ADN acellulaire, constitue un biomarqueur de l'endométriose.

Plus particulièrement, la présente invention porte sur l'utilisation de la DNase pour traiter une endométriose chez une patiente dont l'ADN acellulaire présente un profil de méthylation tel que :
(i) un ou plusieurs gènes sélectionnés dans le groupe constitué par les gènes CALD1, RRP1, FN1, FAM87B, TCEAL6, RPL29P2, ATP11A-AS1, DIP2C, SLCO2B1 et RMI2 est hyperméthylé, et/ou
(ii) un ou plusieurs gènes sélectionnés dans le groupe constitué par les gènes FBX038, ACVR2A, USP1, TDRD5 et STAU2-AS1 est hypométhylé.

Par « hyperméthylation d'un gène » (ou « hypométhylation d'un gène »), on entend ici un niveau de méthylation supérieur (ou inférieur) au niveau taux de normométhylation de 15% de la séquence codante du gène, entraînant une augmentation (ou une répression) de l'expression de ce gène.

Bien évidemment, l'homme du métier peut enrichir et/ou affiner le profil de méthylation de l'ADN acellulaire permettant d'identifier les patientes qui répondront le plus favorablement à un traitement selon l'invention.

L'homme du métier peut notamment, par des travaux de routine, mesurer le différentiel de méthylation de tout ou partie des gènes mentionnés ci-dessus (signature de 15 gène ou également la liste de 36 gènes hyperméthylés et de 26 gènes hypométhylés mentionnée au deuxième paragraphe de la description détaillée) dans différentes cohortes (patientes souffrant d'endométriose ayant ou non répondu au traitement par la DNase) et établir ainsi, en fonction de la technologie utilisée pour la mesure de ces taux de méthylation, des profils de référence auxquels sera comparé le profil de la patiente. Le cas échéant, l'homme du métier peut établir des profils correspondant à différentes formes d'endométriose, en effectuant ces mesures de routine dans différentes cohortes de patientes présentant des formes plus ou moins graves d'endométriose et ayant, ou non, répondu au traitement par la DNase. Le profil de la patiente sera ensuite comparé à ces différents profils, ce qui permettra de traiter spécifiquement les patientes susceptibles de répondre favorablement au traitement.

Pour identifier les patientes les plus à même de répondre au traitement selon l'invention, le profil de méthylation de leur ADN acellulaire peut être mesuré par n'importe quel procédé décrit dans la littérature scientifique.

En particulier, trois grandes méthodes moléculaires, fondées sur une détection enzymatique, immunologique ou chimique, permettent de cartographier les cytosines méthylées. Dans le cadre de la présente invention, ces différentes techniques peuvent être combinées à des méthodes d'hybridation sur puces ou de séquençage à haut débit pour une résolution plus détaillée. Les quatre techniques les plus couramment utilisées sont MeDIP-seq, WGBS, RRBS et 450K Bead Array. Ces différentes méthodes peuvent être aisément mises en œuvre par l'homme du métier, grâce à la disponibilité de protocoles détaillés dans la littérature, de kits commerciaux et de laboratoires spécialisés. Ces différentes méthodes produisent des résultats concordants, avec cependant des sensibilités variables de détection de régions différentiellement méthylées entre échantillons. Aussi, dans le cadre de la présente invention, le niveau « normal » de méthylation pour les gènes analysés doit être calibré en utilisant la technique qui sera utilisée pour mesurer le niveau de méthylation des gènes à partir de l'échantillon biologique.

### Méthode de détection immunologique

Les anticorps spécifiques des cytosines méthylées permettent une détection par immunoprécipitation, selon une méthode appelée MeDIP (*Methylated DNA ImmunoPrecipitation*). Classiquement, l'ADN est fragmenté par sonication, et les fragments les plus méthylés seront les plus favorablement précipités en présence de l'anticorps, permettant d'obtenir une fraction du génome enrichie en méthylation. Dans le cadre de la présente invention, l'étape de sonication n'est pas indispensable, étant donné le caractère fragmenté de l'ADN acellulaire. Couplée au séquençage à haut débit (MeDIP-seq), cette méthode permet de mesurer une densité locale de méthylation, avec une résolution d'environ 200 nucléotides correspondant à la taille moyenne des fragments, avec un coût raisonnable. Elle autorise une couverture complète du génome, avec cependant un biais pour les régions les plus riches en motifs CpG.

### Méthodes de détection chimique

Le seul outil permettant d'interroger le statut de méthylation à l'échelle de la cytosine individuelle est fondé sur le bisulfite. En présence de ce composé chimique, les cytosines sont converties en uracile, alors que les cytosines méthylées ne sont pas affectées. Cette méthode permet ainsi une lecture de la méthylation par analyse des polymorphismes nucléotidiques simples (SNP), dans lesquels un T correspond à une cytosine non modifiée et un C à une cytosine méthylée sur le génome de référence avant conversion.

Le séquençage complet de l'ADN après traitement au bisulfite, ou WGBS (*Whole-Genome Bisulfite Sequencing*), permet d'accéder au statut de méthylation de toutes les cytosines, représentant l'excellence de toutes les méthodes de cartographie de la méthylation génomique.

Le RRBS (*Reduced Representation Bisulfite Sequencing*) est une technique dérivée du WGBS, fondée sur la sélection préalable des régions génomiques riches en CpG par l'utilisation d'enzymes de restriction. En réduisant le nombre de fragments à séquencer, le coût et la profondeur du séquençage s'en trouvent grandement améliorés, dans le même ordre de grandeur que le MeDIP-seq.

Enfin, l'ADN converti au bisulfite peut aussi être hybridé sur une puce d'oligonucléotides, comprenant des oligonucléotides spécifiques des gènes différentiellement méthylés chez les patients endométriosiques.

### Mode d'administration de la DNase

Lorsqu'elle est administrée pour traiter l'encombrement bronchique, cette DNase est normalement administrée par inhalation d'un aérosol produit par un système de nébulisation à air comprimé. Toutefois, ce mode d'administration ne permet pas - sauf éventuellement à augmenter les doses administrées - d'obtenir une concentration plasmatique suffisante pour avoir un effet important sur la concentration d'ADN acellulaire circulant. En outre, l'étude toxicologique de Pulmozyme^{®} a montré que ce produit ne présentait pas d'effets secondaires lorsqu'il était administré par d'autres voies, y compris par voie intraveineuse. Aussi, dans le cadre de la présente invention, la DNase est préférentiellement administrée par voie intramusculaire. Bien entendu, l'homme du métier peut choisir, sur la base de ses connaissances générales en galénique, tout autre mode d'administration permettant d'obtenir une concentration de DNase efficace au plan thérapeutique.

### Posologie et protocoles thérapeutiques

Dans les expériences rapportées ci-dessous, les inventeurs ont observé une nette amélioration clinique chez des patientes souffrant d'endométriose traitées pendant un mois par injection intra musculaire (IM) de DNase I, à la dose de 2500 UI tous les deux jours. En particulier, plusieurs patientes ont constaté une amélioration de leur qualité de vie liée à une diminution de la douleur et de la dyspareunie.

Au plan biologique, les inventeurs ont observé une réduction importante des taux d'ADN libre.

Ces résultats apportent la preuve de l'efficacité de la DNase dans le traitement de l'endométriose, en particulier chez les patientes présentant des taux élevés d'ADN acellulaire, et ouvrent la voie à une nouvelle prise en charge de cette maladie.

Selon une mise en œuvre particulière de l'invention, la DNase est administrée pour traiter l'endométriose à une dose d'au moins 2500 UI tous les deux jours, pendant au moins deux semaines, de préférence au moins trois semaine, et de manière encore préférée pendant un mois ou davantage.

Selon une autre mise en œuvre de l'invention, une dose deux fois supérieure est administrée aux patientes. Par exemple, une dose de 5000UI peut être administrée tous les deux jours, ou la DNase peut être administrée à une dose quotidienne de 2500 UI.

Selon une autre mise en œuvre de l'invention, une dose de 2500 UI tous les jours ou tous les deux jours est administrée, jusqu'à ce qu'une amélioration significative des symptômes soit observée (par exemple, jusqu'à ce que la douleur ait diminué de façon importante) et/ou jusqu'à ce que le niveau d'ADN acellulaire soit descendu en-deçà d'un seuil prédéterminé (correspondant, par exemple, à un niveau proche du niveau observé en moyenne chez les sujets non atteints d'endométriose).

Selon une autre mise en œuvre de l'invention, une dose de 2500 UI tous les jours ou tous les deux jours est administrée pendant une durée déterminée initialement, par exemple pendant un, deux ou trois mois. L'homme du métier pourra bien évidemment ajuster cette durée selon le niveau de gravité de l'endométriose et/ou d'autres paramètres tels que, par exemple, le taux d'ADN acellulaire de la patiente au début du traitement.

Bien entendu, l'homme du métier est également capable, à l'aide de ses connaissances générales notamment en matière de galénique, d'identifier des modes d'administrations et/ou des formes galéniques à libération prolongée, permettant d'espacer les injections tout en conservant au cours du temps une concentration plasmatique de DNase satisfaisante.

Différents protocoles thérapeutiques sont envisagés dans le cadre de la présente invention, dépendant notamment du niveau de gravité de l'endométriose.

Ainsi, pour les stades les moins graves (endométriose de stade I ou II), l'endométriose peut être traitée uniquement par l'administration de DNase comme cela a été décrit plus haut, tandis que pour les formes plus sévères (endométriose de stade III ou IV), le traitement par la DNase pourra avantageusement être complété par une chirurgie.

Selon une autre mise en œuvre de l'invention, le traitement comporte plusieurs phases. Dans une première phase, dite « phase d'attaque », la patiente recevra de la DNase à une dose assez élevée, par exemple 2500 UI par jour. Cette phase d'attaque peut avoir une durée fixe (par exemple de 1 à 3 mois), ou une durée déterminée par l'évolution clinique de la maladie ou encore par un marqueur biologique (tel que le taux d'ADN acellulaire plasmatique).

A l'issue de cette phase d'attaque, et afin d'améliorer le résultat et/ou d'éviter une récidive, le traitement sera poursuivi par un traitement d'entretien, qui sera choisi par le praticien en fonction de différents facteurs. Par exemple, pour une patiente ayant un projet de grossesse, le traitement à la DNase sera poursuivi, le cas échéant en choisissant une dose ou une fréquence plus faible que pendant le traitement d'attaque. A titre d'exemple, on peut envisager une administration de 2500 UI une ou deux fois par semaine, voire toutes les deux semaines ou même une fois par mois. Pour une patiente n'ayant pas de projet de grossesse, le traitement d'entretien peut être soit la poursuite du traitement par DNase (dans les mêmes conditions que pour une patiente ayant un désir de grossesse), soit un traitement hormonal destiné à supprimer les règles (contraceptifs œstroprogestatifs monophasiques en continu, progestatifs, Danazol, analogues ou antagoniste du GnRH).

La présente invention est davantage illustrée dans la partie expérimentale ci-dessous, qui n'en limite pas la portée.

### EXEMPLES

### Introduction

Dans une étude sur un groupe de 32 femmes (16 sans antécédent d'endométriose et 16 avec des antécédents d'endométriose, traitées médicalement et/ou chirurgicalement), les inventeurs ont mis en évidence qu'en moyenne, le groupe endométriose contenait 56% de plus d'ADN libre que le groupe contrôle.

En comparant le cfDNA de 5 femmes de chacun des deux groupes par une étude génomique complète (WGS) en analysant, pour chacun des gènes identifiés, son statut de méthylation, ils ont ensuite observé des différences significatives du niveau de méthylation de certains gènes.

En effet, les inventeurs ont montré que : (i) une hyperméthylation des gènes sélectionnés parmi CALD1, RRP1, FN1, FAM87B, TCEAL6, RPL29P2, ATP11A-AS1, DIP2C, SLCO2B1, RMI2, MIR3170, LINC01007, TSPAN17, MIR4693, HYOU1, TLR4, ADGRL3, IL6, VIRMA, MKRN1, INSIG1, ROR2, MRPL3, FMNL2, TMEM19, ZNF438, LINC01192, RCBTB1, TSPAN33, NKD2, FGFR2, TPRG1, MIR4644, FOXO4, FSTL1 et CLMN, et/ou (ii) une hypométhylation des gènes sélectionnés parmi STAU2-AS1, TDRD5, USP1, ACVR2A, FBXO38, NT5C2, NAV1, SOD3, C3, UBE3A, MIR4655, MYO5C, COX6C, MIR6133, BRSK2, MIR4277, MIR4251, MN1, MIR3666, AZIN1, MIR4251, SLC37A2, FZD10, FASN, MKRN9P et PCCA-AS1, constituent des marqueurs d'une endométriose dont le potentiel évolutif est d'autant plus important que l'hyper- ou hypo-méthylation mesurée est importante.

Plus particulièrement, les inventeurs ont identifié une liste de 15 gènes fortement impliqués dans l'endométriose (10 hyperméthylés et 5 hypométhylés), à savoir: CALD1, RRP1, FN1, FAM87B, TCEAL6, RPL29P2, ATP11A-AS1, DIP2C, SLCO2B1, RMI2, FBX038, ACVR2A, USP1, TDRD5 et STAU2-AS1.

Ces résultats ont été décrits en détails dans la demande de brevet PCT/EP2020/069015, non publiée avant le dépôt de la présente demande.

Poursuivant leurs recherches en vue de traiter les patientes atteintes d'endométriose, les inventeurs ont obtenu les résultats expérimentaux ci-dessous.

### Exemple 1 : Amélioration clinique chez des patientes atteintes d'endométriose traitées par de la DNase I

### Matériels et Méthodes

### Sélection des patientes

- Neuf patientes jeunes, âgées de 26 à 39 ans, algiques, infertiles primaires, dont l'endométriose a été formellement attestée par laparoscopie ont accepté de bénéficier pendant un mois d'un traitement par une DNase I exogène.
- Les patientes souffraient toutes de douleurs abdomino-pelviennes chroniques, de dyspareunies, et de saignements intempestifs pendant leurs cycles menstruels.
- Toutes avaient bénéficié d'une laparoscopie pour confirmer le diagnostic d'endométriose.
- Elles présentaient une Endométriose pelvienne de stade II fort au minimum ou plus (Stade III et IV) ; schématiquement, le stade II est localisé au péritoine et/ou aux ligaments utéro-sacrés ou aux trompes ; le stade III associe les ovaires et le stade IV l'appareil digestif et particulièrement la cloison recto-vaginale.
- Les patientes n'avaient aucun traitement médical en cours depuis plus de 3 mois et ne devaient pas prendre d'antalgique pendant leur traitement par la DNase.
- Les femmes participant à l'étude étaient aptes à recevoir les informations quant au traitement et au suivi du traitement.
- Toutes étaient jugées capables d'évaluer leur douleur et de communiquer avec leur médecin pour l'analyse de leur confort de vie.

Chacune d'elle a signé un formulaire de consentement après une information de la nature et des modalités du traitement et accepté une évaluation biologique.

Le comité d'éthique de l'Université de Sousse a donné son accord au projet thérapeutique et au suivi bioclinique des patientes.

### Traitement

Une injection intra musculaire (IM) de 1 ampoule de 2500 UI de DNase I (Pulmozyme ; Laboratoire Roche France, Neuilly sur Seine) tous les deux jours pendant un mois.

La solution pour inhalation Pulmozyme^{®} (dornase alfa) est une solution stérile, limpide, incolore et hautement purifiée de désoxyribonucléase I humaine recombinante (rhDNase) qui clive sélectivement l'ADN.

Pulmozyme^{®} est normalement administré par inhalation d'un aérosol produit par un système de nébulisation à air comprimé. Mais les niveaux systémiques de rh DNase sont très faibles (maximum 15%) après inhalation ; il a donc été décidé d'administrer ce même produit par injection IM afin d'augmenter la concentration de DNase, ayant bien noté qu'il n'y avait pas d'effets secondaires dans l'étude toxicologique de Pulmozyme, y compris par voie intraveineuse.

Le choix de 2500UI tous les deux jours nous a été dicté par la volonté de baisser les taux d'ADN libre à un niveau habituellement rencontré chez des femmes sans endométriose ni autre pathologie inflammatoire.

La durée d'un mois de traitement est apparue nécessaire pour diminuer drastiquement le stress oxydatif et l'inflammation.

Pendant la durée du traitement une consultation régulière permettait d'évaluer l'intensité de la douleur et le confort de vie des patientes.

### Qualité de vie : les instruments d'évaluation

Parmi les divers questionnaires à disposition, les patientes ont été soumises aux 8 items du PWI créé en 2018 par Rush et al. (Personnal Well Being Index) portant sur le confort de vie, les réalisations professionnelles, les relations sociales, la sécurité, les communications communautaires, la vision du futur, satisfaction de la vie globale, sur une échelle de 0 à10 (de totalement insatisfaite à complètement satisfaite).

Pour évaluer la douleur, l'échelle VAS (*Visual Analogue Scale*) a été utilisée pour cinq composantes telles que la dysménorrhée, la dyspareunie, la dyschésie, les douleurs pelviennes chroniques et la dysurie sur une échelle horizontale de 10 cm allant de l'absence totale de douleurs aux douleurs insupportables.

### Résultats cliniques

### Influence sur la douleur

- Sept des neuf patientes traitées ont constaté une diminution de leurs douleurs. Les deux autres n'ont pas pu être évaluées pour non respect de la procédure.
- Six patientes sur sept ont déclaré avoir eu une diminution des douleurs quel que soit le stade de l'endométriose.

5 sont passés de 9 à 4 de cotation sur l'échelle VAS et 1, âgée de 38 ans, stade III de 8 à 4.

Une patiente, la plus jeune, âgée de 26 ans, endométriose de stade II fort, n'a pas eu de diminution significative des douleurs

Il est connu qu'une intervention chirurgicale sur tous types d'endométriose ne supprime pas systématiquement la douleur. Cette dernière relève aussi du contexte inflammatoire, du saignement des foyers d'endomètre ectopiques et d'une activation nerveuse ou d'une activation altérée des voies nociceptives.

La réaction inflammatoire s'accompagne en effet de sécrétion locale de cytokines (IL6, TNF alpha, NGF) et autres prostaglandines (PGE2) responsables de contractions utérines, de saignements et de douleurs.
- Six patientes éligibles ont déclaré avoir eu une amélioration de leur dyspareunie; cotations de 8 à 4 sur l'échelle PWI.

L'une d'entre elles a déclaré une disparition totale de la dyspareunie et une amélioration spectaculaire de son confort de vie : cotation de 8 à 1

Une autre âgée de 38 ans, endométriose de stade III, qui a connu une amélioration de la douleur de 50%, n'a noté qu'une amélioration de 10% pour la dyspareunie.

L'influence de l'endométriose sur les rapports sexuels et plus généralement sur le couple est une des données essentielles de cette pathologie.

Aucune différence n'a été observée en fonction du stade de l'endométriose et des localisations de foyers d'endométriose.

Pour les dysfonctions sexuelles, l'interprétation est rendue plus délicate si l'on prend en compte les facteurs psychologiques et autres comorbidités.
- Quatre patientes sur sept présentant habituellement des saignements utéro vaginaux intempestifs sept à dix jours avant les règles, ont déclaré leur disparition totale sous traitement par la Dornase alfa.

Cet effet intra utérin sous-entend la même action au niveau des sites ectopiques participant aussi à la diminution des douleurs abdomino-pelviennes. Ce résultat est très prometteur quant à l'efficacité du traitement.

### Exemple 2 : Effets biologiques du traitement par de la DNase I chez des patientes atteintes d'endométriose

### Matériels et Méthodes

Les analyses présentées ci-dessous ont été réalisées par la plateforme de recherche ACOBIOM à Montpellier.

### Analyse de la méthylation de l'ADN par séquençage

L'expression de certains gènes peut être contrôlée par la méthylation des cytosines dans des îlots di-nucléotidiques CpG de l'ADN. La dérégulation de la méthylation peut entraîner différentes pathologies (maladies neurologiques, cancer, *etc*). La méthode employée ici est par le traitement au bisulfite qui convertit les cytosines non-méthylées en uracile. Les cytosines méthylées sont protégées et sont ainsi facilement identifiées par séquençage (méthode Sanger ou plateforme de type NGS (Next-Gen sequencing)).

### Méthode d'extraction de l'ADN libre sérique

Nous avons utilisé le kit de Qiagen QIAamp DNA Blood Mini Kit Cat No./ID: 51104 suivant le protocole « QIAamp DNA Mini and Blood Mini Handbook » version du 05/2016.

A 400µl de sérum pipeté dans un microtube de 1.5 ml nous avons ajouté 40 µl de Protéase (Protéinase K) à la concentration d'environ 600 mAU/ml ou 40 mAU/mg de protéine et 400 µl de tampon de dénaturation contenant de la guanidine (Tampon AL de Qiagen) puis mélangé au vortex (5 fois 5 secondes) avant d'être incubé à 56°C pendant 10 minutes.

Au mélange précédent, 400 µl d'éthanol non-dénaturé à 100% ont été ajoutés et mélangés au vortex (5 fois 5 secondes). La moitié de ce mélange (420 µl) a ensuite été chargée sur une colonne du kit et centrifugée à 8000 x g pendant 30 secondes. Après avoir éliminé la partie préalablement centrifugée, l'autre moitié (420 µl) a été chargée sur la même colonne et centrifugée à 8000 x g pendant 30 secondes.

L'ADN fixé sur les billes de silica trappées dans la colonne Qiagen a été lavé une fois avec 750 µl de tampon AW1 (tampon contenant de la guanidine et de l'éthanol) puis centrifugé à 8000 x g pendant 30 secondes. Un second lavage avec 750 µl de tampon AW2 (tampon contenant du Tris, du NaCI et de l'éthanol) et une centrifugation à 8000 x g pendant 30 secondes.

La membrane trappant l'ADN a été séchée par centrifugation pendant 1 minute à 14000 x g. L'élution de l'ADN a été effectuée en deux fois avec 25 µl de tampon AE (équivalent au tampon TE : Tris/EDTA 1mM/0.1mM) après incubation de 2 minutes chaque fois et centrifugation à 8000 x g pendant 1 minute chaque fois.

L'ADN a été conservé au froid entre 0°C et 8°C ou congelé à une température comprise entre -16°C et -85°C.

### Méthode de mesure de la concentration d'ADN libre

### Matériel

- Séquence des amorces utilisées pour amplifier une région de 86 paires de bases à cheval sur l'exon 1 et l'intron 2 du gène *Homo sapiens* ribonuclease P/MRP subunit p30, abrégé RPP30 (NM_006413, ENST00000371703.7) : RNP30_F : AGATTTGGACCTGCGAGCG (SEQ ID No : 1) et RNP30_R : GAAGCCGGGGCAACTCAC (SEQ ID No : 2)
- Thermocycleur en temps réel type LightCycler 480II.
- Master mix contenant du SYBRgreen Type LightCycler^{®} 480 SYBR Green I Master (Cat. no 04707516001)

### Méthode

Un triplicat de 5 µl d'DNA extrait ont été ajoutés à 20 µl de 1 x Master mix contenant 0.5 µM de chaque amorce. L'amplification consiste en une activation de 5 minutes à 95°C puis 35 cycles de dénaturation à 95°C pendant 10 secondes, d'hybridation à 59°C pendant 20 secondes, d'élongation à 72°C pendant 15 secondes suivie d'une élongation finale de 5 minutes à 72°C.

Le calcul de la concentration se fait par régression linéaire avec une échelle de dilution de 10 fois, en triplicat, d'un ADN humain de concentration connue (en partant de 500'000 copies/réaction à 1'000 copies par réaction) et comparaison des Ct (*Cycle threshold*) selon les méthodes connues et décrites pour la PCR quantitative (qPCR). La longueur du génome humain haploïde considéré est de 3.2 x 10⁹ paires de base avec une masse molaire de 650 g/mole/pb ce qui nous donne 290 copies/ng d'ADN haploïde.

Un exemple de résultats est présenté dans le tableau ci-dessous :

**Tableau**

| **ID patiente envoi 1** | **Concentration ADN (ng/µl)** |
|---|---|
| **A** | 4,1 |
| **B** | 1,4 |
| **C** | 4,2 |
| **D** | 1,7 |
| **E** | 5,2 |
| **F** | 0,5 |
| **G** | 2,8 |
| **H** | 0,4 |
| **I** | 1,9 |
| **J** | 0,3 |
| **K** | 2,5 |
| **L** | 4 |
| **M** | 0,6 |
| **N** | 3,1 |

### Traitement des ADN extrait au bisulfite

La totalité des 45µl de chacun des échantillons sont traités au bisulfite à l'aide du kit EZ DNA Methylation Kit de Zymo Research selon le protocole fournisseur. Les cytosine méthylées seront protégées et ne seront pas converties, alors que les cytosines non méthylées seront converties en uracile (U). Le volume d'élution à la fin de la procédure de conversion est de 25µl.

### Contrôle de la concentration des ADN traités

La concentration des échantillons traités au bisulfite est mesurée à l'aide du spectrophotomètre Nano-Drop ND-1000 de Thermo Scientific. 1µl est utilisé pour réaliser la mesure.

Exemple de résultats dans le tableau suivant :

| **ID échantillons** | **Concentration ADN (ng/µl)** | **Absorbance ratio 260/280** | **Absorbance ratio 260/230** | **Quantité ADN dans 20 µl** |
|---|---|---|---|---|
| **A** | 10,57 | 1,4 | 0,98 | 211,4 |
| **B** | 9,23 | 1,26 | 0,58 | 184,6 |
| **C** | 24,16 | 1,49 | 1,05 | 483,2 |
| **D** | 8,31 | 1,32 | 0,72 | 166,2 |
| **E** | 16,42 | 1,6 | 1,21 | 328,4 |
| **F** | 5,84 | 1,03 | 0,65 | 116,8 |
| **G** | 6,78 | 1,61 | 0,77 | 135,6 |
| **H** | 3,91 | 1,36 | 0,54 | 78,2 |
| **I** | 6,73 | 1,19 | 0,67 | 134,6 |
| **J** | 4,04 | 0,99 | 0,39 | 80,8 |
| **K** | 11,44 | 1,37 | 0,33 | 228,8 |
| **L** | 14,45 | 2,11 | 1,76 | 289 |
| **M** | 6,4 | 1,23 | 0,88 | 128 |
| **N** | 6,92 | 1,49 | 0,87 | 138,4 |

### Réactions de PCR

### • Design des amorces PCR

15 cibles ont été identifiées lors de précédentes expérimentations (Cf. tableau ci-dessous :

| **feature.name** | **feature.desc** | **feature.id** |
|---|---|---|
| CALD1 | caldesmon 1 | NM_033140 |
| RRP1 | ribosomal RNA processing 1 | NM_003683 |
| FN1 | fibronectin 1 | NM_212482 |
| FAM87B | family with sequence similarity 87 member B | NR_103536 |
| TCEAL6 | transcription elongation factor A like 6 | NM_001006938 |
| RPL29P2 | ribosomal protein L29 pseudogene 2 | NR_002778 |
| ATP11A-AS1 | ATP11A antisense RNA 1 | NR_046661 |
| DIP2C | disco interacting protein 2 homolog C | NM_014974 |
| SLCO2B1 | solute carrier organic anion transporter family member 2B1 | NM_007256 |
| RMI2 | RecQ mediated genome instability 2 | NM_152308 |
| STAU2-AS1 | STAU2 antisense RNA 1 | NR_038406 |
| TDRD5 | tudor domain containing 5 | NM_001199091 |
| USP1 | ubiquitin specific peptidase 1 | NM_003368 |
| ACVR2A | activin A receptor type 2A | NM_001616 |
| FBXO38 | F-box protein 38 | NM_001271723 |

Pour chacune des 15 cibles un couple d'amorces PCR spécifique a été défini. En gras sont indiquées les bases modifiées : Y (base T ou C) et R (base A ou G).

| Amorce | SEQ ID No : | Séquence | Taille de l'amplicon (pb) |
|---|---|---|---|
| **CALD1_F** | 3 | AAGACTCGGCAGCATCTCCATGGGAGTTATTYGTGGGTTT | 179 |
| CALD1 R | 4 | GCGATCGTCACTGTTCTCCACCCCTTCCCTCACCAATAAA | |
| **RRP1_F** | 5 | AAGACTCGGCAGCATCTCCAGYAATGGGYATGTTAGGYTT | 201 |
| RRP1 R | 6 | GCGATCGTCACTGTTCTCCACCCACCCTATTCTCARCARC | |
| **FN1_F** | 7 | AAGACTCGGCAGCATCTCCATTTTTYGTTTAGTTTYGATGTAGGT | 223 |
| FN1-R | 8 | GCGATCGTCACTGTTCTCCACACATAAACTTTCCTTTTCCCCTCA | |
| **FAM87B_F** | 9 | AAGACTCGGCAGCATCTCCAGTAGGTAGTGTAGATAGYGTG | 236 |
| FAM87B-R | 10 | GCGATCGTCACTGTTCTCCATATCTACACTACCTRCCTAATC | |
| **TCEAL6_F** | 11 | AAGACTCGGCAGCATCTCCATGGGGTATATGGGYTTTTGAAATTAA | 220 |
| TCEAL6_ R | 12 | GCGATCGTCACTGTTCTCCAAACTCCAAACTATTCCCTTCTCCA | |
| **RPL29P2_F** | 13 | AAGACTCGGCAGCATCTCCATGYGTTYGGTTTTATTTTAGGAA | 162 |
| RPL29P2_R | 14 | GCGATCGTCACTGTTCTCCATGYGTTYGGTTTTATTTTAGGAA | |
| **ATP11A-AS1_F** | 15 | AAGACTCGGCAGCATCTCCAAGTTTAGGATTTTAGAGYGTGAGTG | 231 |
| ATP11A-AS1_R | 16 | GCGATCGTCACTGTTCTCCACCCCAACCAACAACRAAACC | |
| **DIP2C_F** | 17 | AAGACTCGGCAGCATCTCCAGGGGTTGTGATAYGTTGTTTTGA | 202 |
| DIP2C_ R | 18 | GCGATCGTCACTGTTCTCCAACRTCTATCCTAATCCTACCACTCA | |
| **SLCO2B1_F** | 19 | AAGACTCGGCAGCATCTCCATGYTGGTTAAGTGATTTTAGGYA | 229 |
| SLCO2B1 R | 20 | GCGATCGTCACTGTTCTCCACTCTAARCTCCAARCCTRCA | |
| **RMI2_F** | 21 | AAGACTCGGCAGCATCTCCAGGAGGTGATGYGATGGYTTA | 235 |
| RMI2 R | 22 | GCGATCGTCACTGTTCTCCACCCACTRCCTCACTCTCATC | |
| **STAU2-AS1_F** | 23 | AAGACTCGGCAGCATCTCCAAGATGGTTTTGGGTAGGAGAAAA | 216 |
| STAU2-AS1_R | 24 | GCGATCGTCACTGTTCTCCATACACCTCCCAAAACRACCC | |
| **TDRD5_F** | 25 | AAGACTCGGCAGCATCTCCAGGYGGGGGTAGGTAGTTTTY | 229 |
| TDRD5_ R | 26 | GCGATCGTCACTGTTCTCCACACRCRCTCCAACAACTAAT | |
| **USP1_F** | 27 | AAGACTCGGCAGCATCTCCAAGGTGGGATAGTAAAATTGAGTAGA | 221 |
| USP1 R | 28 | GCGATCGTCACTGTTCTCCAAACRCCAACRAACACACAAC | |
| **ACVR2A_F** | 29 | AAGACTCGGCAGCATCTCCATGTGTTTTAAGGGTTTGGGGT | 180 |
| ACVR2A-R | 30 | GCGATCGTCACTGTTCTCCAACCTTTACACTTTTCATACRCCT | |
| **FBXO38_F** | 31 | AAGACTCGGCAGCATCTCCATGGTTTTTAGATGATTTATGTTGGAGT | 180 |
| FBXO38_R | 32 | GCGATCGTCACTGTTCTCCAACCCAACACTTAACTTCACCT | |

### • Mélange réactionnel de PCR

Les réactions de PCR sont réalisées dans un volume final de 25µl contenant :

| | |
|---|---|
| Taq'Ozyme HS mix 2X | 12,5µl |
| Amorces Sens + Antisens 2µM | 5µl (0,4µM final) |
| H₂O pure | 6,5µl |
| Matrice ADN traité au Bisulfite | 1ng/µl 1µl |

### • Programme PCR

| | |
|---|---|
| 95°C | 1 minute |
| 95°C | 15 secondes |
| 56°C | 15 secondes |
| 72°C | 30 secondes |
| X 45 cycles | |
| 72°C | 5 minutes |

Les produits PCR générés sont vérifiés par électrophorèse en agarose 1% (3µl) et sont ensuite séquencés. Les cytosines qui étaient initialement méthylées ont été « protégées » et n'ont pas été converties lors du traitement bisulfite, alors que les cytosines non méthylées seront converties en uracile (U). Ainsi, au séquençage, une cytosine méthylée apparaitra comme une cytosine (C) alors qu'une cytosine non méthylée apparaitra comme une Thymine (T).

### Résultats biologiques

### Influence du traitement sur les taux d'ADN libre

L'extraction des ADN libres sériques a montré une diminution de 40% de diminution des taux en fin de traitement, comparés aux taux avant traitement.

Les taux d'ADN libre très élevés des 7 patients éligibles avant traitement ont été réduits de près de la moitié avec 2500 UI de Dornase I tous les deux jours. Ceci suggère un effet dose et la possibilité de réduire encore les taux d'ADN circulants en administrant 2500 UI par jour pour une meilleure efficacité.

### Le profil génique et de méthylation

Les sept ADN sériques avant traitement et les sept après traitement, sont soumis à un WGS (*Whole Genome Sequencing*), afin de déterminer, notamment, le statut de méthylation des gènes candidats (10 hyperméthylés et 5 hypométhylés) cités dans l'introduction de cette partie expérimentale.

L'obtention des résultats de cette analyse a été retardée par la pandémie de Covid19. Néanmoins, il est anticipé que le profil de méthylation de ces gènes sera sensiblement perturbé après traitement par la Dornase alfa et la répartition génique, relativement inchangée, laissera apparaître de nouvelles données dans la distribution.

L'étude de networks devrait refléter une nouvelle répartition des voies métaboliques témoignant de l'effet bénéfique du traitement.

### Références

Ahn SH, Singh V, Tayade C. Biomarkers in endometriosis: challenges and opportunities. Fertil Steril. 2017 ; 107(3):523-532.
Blumenkrantz MJ, Gallagher N, Bashore RA, Tenckhoff H, 1981. Retrograde menstruation in women undergoing chronic peritoneal dialysis. Obstet Gynecol; 57:667-70.
Della Corte , Di Filippo C , Gabrielli O , Reppuccia S , La Rosa V L , Ragusa R, Fichera M , Commodari E , Bifulco G, Giampaolino P. The Burden of Endometriosis on Women's Lifespan: A Narrative Overview on Quality of Life and Psychosocial Wellbeing. Int. J. Environ. Res. Public Health 2020, 17, 4683.
Filev AD, Shmarina GV, Ershova ES, Veiko NN, Martynov AV, Borzikova MA, Poletkina AA, Dolgikh OA, Veiko VP, Bekker AA, Chirkov AV, Volynshchikov ZN, Deviataikina AS, Shashin DM, Puretskiy VK, Tabakov VJ, Izhevskaya VL, Kutsev SI, Kostyuk SV, Umriukhin PE. Oxidized Cell-Free DNA Role in the Antioxidant Defense Mechanisms under Stress. Oxid Med Cell Longev. 2019; 8; 2019:1245749.
Grabuschnig S, Bronkhorst AJ, Holdenrieder S, Rosales Rodriguez I, Schliep KP, Schwendenwein D, Ungerer V, Sensen CW. Putative Origins of Cell-Free DNA in Humans: A Review of Active and Passive Nucleic Acid Release Mechanisms*.*
Halme J,Hammond MG, Hulka JF, Raj SG, Talbert LM, 1984.Retrograde menstruation in healthy women and in patients with endometriosis. Obstet Gynecol; 64:151-4.
Hazout A, Montjean D, Cassuto NG, Belloc S, Dalleac A, Tesarik J and Benkhalifa M, 2018. Free Circulating Nucleic Acids and Infertility. JGWH.
Koninckx PR, Ussia A, Adamyan L, Wattiez A, Gomel V, Martin DC. Pathogenesis of endometriosis: the genetic/epigenetic theory. Fertil Steril. 2019; 111(2):327-340.
Laganà AS, Garzon S, Götte M, Viganô P, Franchi M, Ghezzi F, Martin DC. The Pathogenesis of Endometriosis: Molecular and Cell Biology Insights. Int J Mol Sci. 2019 Nov 10; 20(22):5615.
Liu K, Zhang W, Liu S, Dong B, Liu Y, 2015. Hepatic endometriosis: a rare case and review of the literature. Eur J Med Res; 20:48.
Mandel P, Metais P, 1948. Les acides nucléiques du plasma sanguin chez l'homme. CR Seance Soc Biol Ses Fil. 142 : 241-243.
Marsh E, Marc R Laufer ; Endometriosis in premenarcheal girls who do not have an associated obstructive anomaly. Fertil Steril. 2005 Mar;83(3):758-60.
McPeak AE, Allaire C, Williams C, Albert A, Lisonkova S, Yong PJ. Pain catastrophizing and pain Health - Related Life Clin J Pain. 2018; 34(4):349-356.
Naqvi H, Ilagan Y, Krikun G, Taylor HS, 2014. Altered genome-wide methylation in endometriosis. Reprod Sci; 21:1237-43.
Nasu K, Kawano Y, Tsukamoto Y, Takano M, Takai N, Li H, Furukawa Y, Abe W, Moriyama M, Narahara H.J. Aberrant DNA methylation status of endometriosis: epigenetics as the pathogenesis, biomarker and therapeutic target. Obstet Gynaecol Res. 2011 Jul; 37(7): 683-95.
Rei C, Williams T, and M Feloney, 2018. Endometriosis in a Man as a Rare Source of Abdominal Pain: A Case Report and Review of the Literature, Case Reports in Obstetrics and Gynecology, vol. 2018, Article ID 2083121.
Rush, G.; Misajon, R. Examining subjective wellbeing and health-related quality of life in women with endometriosis. Health Care Women Int. 2018, 39, 303-321.
Vassilopoulou L, Matalliotakis M, Zerviou M, atalliotaki C, Krithinakis K, Matalliotakis I, Spandidos D, Goulielmos G.N. Defining the genetic profile of endometriosis. Review 2019. Experimental and Therapeutic Medicine.
Zachariah R, Schmid S, Radpour R, Buerki N, Xiu-Cheng Fan A, Hahn S, Holzgreve W, Zhong XY, 2009. Circulating cell-free DNA as a potential biomarker for minimal and mild endometriosis. RBMonline: 407-411.

## Revendications

1. DNase, pour son utilisation dans le traitement de l'endométriose.

2. DNase selon la revendication 1, pour une utilisation selon la revendication 1, ladite DNase étant une DNase recombinante.

3. DNase selon la revendication 1 ou la revendication 2, pour une utilisation selon la revendication 1, ladite DNase étant la DNase I.

4. DNase selon l'une quelconque des revendications 1 à 3, pour une utilisation dans le traitement de l'endométriose chez une patiente présentant un taux d'ADN acellulaire statistiquement supérieur au taux d'ADN libre chez les femmes n'ayant pas d'endométriose.

5. DNase selon l'une quelconque des revendications 1 à 3, pour une utilisation selon la revendication 4, **caractérisée en ce que** l'ADN acellulaire de la patiente présente un profil de méthylation tel que :
(i) un ou plusieurs gènes sélectionnés dans le groupe constitué par les gènes CALD1, RRP1, FN1, FAM87B, TCEAL6, RPL29P2, ATP11A-AS1, DIP2C, SLCO2B1 et RMI2 est hyperméthylé, et/ou
(ii) un ou plusieurs gènes sélectionnés dans le groupe constitué par les gènes FBX038, ACVR2A, USP1, TDRD5 et STAU2-AS1 est hypométhylé.

6. DNase selon l'une quelconque des revendications 1 à 3, pour une utilisation selon l'une quelconque des revendications 1, 4 et 5, **caractérisée en ce que** la DNase est administrée par voie intramusculaire.

7. DNase selon l'une quelconque des revendications 1 à 3, pour une utilisation selon l'une quelconque des revendications 1 et 4 à 6, **caractérisée en ce que** la DNase est administrée à une dose d'au moins 2500 UI tous les deux jours, pendant au moins deux semaines.

8. DNase selon l'une quelconque des revendications 1 à 3, pour une utilisation selon la revendication 7, **caractérisée en ce que** la DNase est administrée à une dose quotidienne de 2500 UI.

9. DNase selon l'une quelconque des revendications 1 à 3, pour une utilisation selon la revendication 7 ou la revendication 8, **caractérisée en ce que** la DNase est administrée à une dose de 2500 UI tous les jours ou tous les deux jours, pendant 1 à 3 mois.

10. DNase selon l'une quelconque des revendications 1 à 3, pour une utilisation selon l'une quelconque des revendications 1 et 4 à 9, pour traiter une patiente atteinte d'endométriose de stade I ou II.

11. DNase selon l'une quelconque des revendications 1 à 3, pour une utilisation selon l'une quelconque des revendications 1 et 4 à 9, pour traiter une patiente atteinte d'endométriose de stade III ou IV, en association avec une chirurgie.

12. DNase selon l'une quelconque des revendications 1 à 3, pour une utilisation selon l'une quelconque des revendications 1 et 4 à 11, **caractérisée en ce qu'**après un premier traitement d'attaque selon l'une quelconque des revendications 7 à 9, la patiente reçoit un traitement d'entretien choisi parmi une hormonothérapie et/ou une administration de DNase à une dose ou une fréquence plus faible que pendant le traitement d'attaque.


SEQUENCE LISTING

<110>  ENDOLIFE

<120>  Utilisation d'une désoxyribonucléase I dans le traitement de
       l'endométriose chez des patientes présentant une signature
       épigénétique de l'endométriose sur l'ADN acellulaire

<130>  B200384EPA

<160>  32

<170>  PatentIn version 3.5

<210>  1
<211>  19
<212>  DNA
<213>  Artificial Sequence

<220>
<223>  primer

<400>  1
agatttggac ctgcgagcg                                                    19


<210>  2
<211>  18
<212>  DNA
<213>  Artificial Sequence

<220>
<223>  primer

<400>  2
gaagccgggg caactcac                                                     18


<210>  3
<211>  40
<212>  DNA
<213>  Artificial Sequence

<220>
<223>  primer

<400>  3
aagactcggc agcatctcca tgggagttat tygtgggttt                             40


<210>  4
<211>  40
<212>  DNA
<213>  Artificial Sequence

<220>
<223>  primer

<400>  4
gcgatcgtca ctgttctcca ccccttccct caccaataaa                              40


<210>  5

<211>  40
<212>  DNA
<213>  Artificial Sequence

<220>
<223>  primer

<400>  5
aagactcggc agcatctcca gyaatgggya tgttaggytt                              40


<210>  6
<211>  40
<212>  DNA
<213>  Artificial Sequence

<220>
<223>  primer

<400>  6
gcgatcgtca ctgttctcca cccaccctat tctcarcarc                              40


<210>  7
<211>  45
<212>  DNA
<213>  Artificial Sequence

<220>
<223>  primer

<400>  7
aagactcggc agcatctcca tttttygttt agtttygatg taggt                        45


<210>  8
<211>  45
<212>  DNA
<213>  Artificial Sequence

<220>
<223>  primer

<400>  8
gcgatcgtca ctgttctcca cacataaact ttccttttcc cctca                        45


<210>  9
<211>  41
<212>  DNA
<213>  Artificial Sequence

<220>
<223>  primer

<400>  9
aagactcggc agcatctcca gtaggtagtg tagatagygt g                            41


<210>  10
<211>  42
<212>  DNA
<213>  Artificial Sequence

<220>
<223>  primer

<400>  10
gcgatcgtca ctgttctcca tatctacact acctrcctaa tc                    42


<210>  11
<211>  46
<212>  DNA
<213>  Artificial Sequence

<220>
<223>  primer

<400>  11
aagactcggc agcatctcca tggggtatat gggyttttga aattaa                46


<210>  12
<211>  44
<212>  DNA
<213>  Artificial Sequence

<220>
<223>  primer

<400>  12
gcgatcgtca ctgttctcca aactccaaac tattcccttc tcca                  44


<210>  13
<211>  43
<212>  DNA
<213>  Artificial Sequence

<220>
<223>  primer

<400>  13
aagactcggc agcatctcca tgygttyggt tttattttag gaa                   43


<210>  14
<211>  43
<212>  DNA
<213>  Artificial Sequence

<220>
<223>  primer

<400>  14
gcgatcgtca ctgttctcca tgygttyggt tttattttag gaa                   43


<210>  15
<211>  45
<212>  DNA
<213>  Artificial Sequence

<220>
<223>  primer

<400>  15
aagactcggc agcatctcca agtttaggat tttagagygt gagtg                        45


<210>  16
<211>  40
<212>  DNA
<213>  Artificial Sequence

<220>
<223>  primer

<400>  16
gcgatcgtca ctgttctcca ccccaaccaa caacraaacc                              40


<210>  17
<211>  43
<212>  DNA
<213>  Artificial Sequence

<220>
<223>  primer

<400>  17
aagactcggc agcatctcca ggggttgtga taygttgttt tga                          43


<210>  18
<211>  45
<212>  DNA
<213>  Artificial Sequence

<220>
<223>  primer

<400>  18
gcgatcgtca ctgttctcca acrtctatcc taatcctacc actca                        45


<210>  19
<211>  43
<212>  DNA
<213>  Artificial Sequence

<220>
<223>  primer

<400>  19
aagactcggc agcatctcca tgytggttaa gtgattttag gya                          43


<210>  20
<211>  40
<212>  DNA
<213>  Artificial Sequence

<220>
<223>  primer

<400>  20
gcgatcgtca ctgttctcca ctctaarctc caarcctrca                              40

<210>  21
<211>  40
<212>  DNA
<213>  Artificial Sequence

<220>
<223>  primer

<400>  21
aagactcggc agcatctcca ggaggtgatg ygatggytta                              40


<210>  22
<211>  40
<212>  DNA
<213>  Artificial Sequence

<220>
<223>  primer

<400>  22
gcgatcgtca ctgttctcca cccactrcct cactctcatc                              40


<210>  23
<211>  43
<212>  DNA
<213>  Artificial Sequence

<220>
<223>  primer

<400>  23
aagactcggc agcatctcca agatggtttt gggtaggaga aaa                          43


<210>  24
<211>  40
<212>  DNA
<213>  Artificial Sequence

<220>
<223>  primer

<400>  24
gcgatcgtca ctgttctcca tacacctccc aaaacraccc                              40


<210>  25
<211>  40
<212>  DNA
<213>  Artificial Sequence

<220>
<223>  primer

<400>  25
aagactcggc agcatctcca ggygggggta ggtagtttty                              40


<210>  26

<211>  40
<212>  DNA
<213>  Artificial Sequence

<220>
<223>  primer

<400>  26
gcgatcgtca ctgttctcca cacrcrctcc aacaactaat                              40


<210>  27
<211>  45
<212>  DNA
<213>  Artificial Sequence

<220>
<223>  primer

<400>  27
aagactcggc agcatctcca aggtgggata gtaaaattga gtaga                        45


<210>  28
<211>  40
<212>  DNA
<213>  Artificial Sequence

<220>
<223>  primer

<400>  28
gcgatcgtca ctgttctcca aacrccaacr aacacacaac                              40


<210>  29
<211>  41
<212>  DNA
<213>  Artificial Sequence

<220>
<223>  primer

<400>  29
aagactcggc agcatctcca tgtgttttaa gggtttgggg t                            41


<210>  30
<211>  43
<212>  DNA
<213>  Artificial Sequence

<220>
<223>  primer

<400>  30
gcgatcgtca ctgttctcca acctttacac ttttcatacr cct                          43


<210>  31
<211>  47
<212>  DNA
<213>  Artificial Sequence

<220>
<223>  primer

<400>  31
aagactcggc agcatctcca tggtttttag atgatttatg ttggagt                    47


<210>  32
<211>  41
<212>  DNA
<213>  Artificial Sequence

<220>
<223>  primer

<400>  32
gcgatcgtca ctgttctcca acccaacact taacttcacc t                          41